# EUROPEAN PATENT APPLICATION

(11) **EP 2 947 077 A1**
(43) Date of publication of application: **25.11.2015**
(21) Application number: 14168813.5
(22) Date of filing: 19.05.2014
(51) Int. Cl.: C07D 309/10, C07D 407/12, C07D 409/10, C07F 1/08

(54) **Stereoselective synthesis of intermediates in the preparation of ß-C-arylglucosides**

(71) Applicant: LEK Pharmaceuticals d.d., 1526 Ljubljana (SI)
(72) Inventor: The designation of the inventor has not yet been filed
(74) Representative: Kluschanzoff, Harald

(57) **Abstract**

The invention belongs to the fields of pharmaceutical industry, and particularly to an improved stereoselective synthesis of intermediate compounds for the preparation of gliflozins, for example canagliflozin or structurally similar gliflozins. Gliflozins, such as canagliflozin, dapagliflozin, empagliflozin, or ipragliflozin, inhibit the sodium-dependent glucose cotransporter 2 (SGLT2) in the kidney and as such are useful in the treatment of type-2 diabetes.

## Description

### Field of the invention

The invention relates to the field of pharmaceutical industry, and particularly to a stereoselective process of suitable intermediate compounds and the intermediate compounds as such, which can be used in the synthesis of sodium-dependent glucose cotransporter 2 (SGLT2) inhibitors ("gliflozins" in the following), for example canagliflozin or structurally similar compounds. Gliflozins, such as canagliflozin, dapagliflozin, empagliflozin, luseogliflozin or ipragliflozin, inhibit the sodium-dependent glucose cotransporter 2 (SGLT2) in the kidney and as such are useful in the treatment of type-2 diabetes.

### Description of the background art

Canagliflozin **(8,** chemical name (2*S*,3*R*,4*R*,5*S*,6*R*)-2-{3-[5-[4-fluoro-phenyl)-thiophen-2-ylmethyl]-4-methyl-phenyl}-6-hydroxymethyl-tetrahydro-pyran-3,4,5-triol) as well as some other molecules of the general formula I, such as dapagliflozin or ipragliflozin, is an inhibitor of the kidney-located sodium-dependent glucose cotransporter 2 (SGLT2) and is indicated in the treatment of type-2 diabetes with the structure shown below.

As indicated by the structure below, canagliflozin exhibits stereoisomerism due to the presence of five chiral centers on the sugar (i.e. glucose) core, wherein the stereoisomer named and shown below is a β-C-arylglycoside (β-C-arylglucoside, respectively), i.e. the aryl moiety on C-1 and the hydroxy group on C-2 are in a *trans*-configuration relative to each other *(trans* isomer in the following). All SGLT2 inhibitors in the market and in advanced clinical phase up till now are *trans*-β-glycosides. Hence, during the manufacturing process of canagliflozin and related compounds, there is a challenge that mixtures of different isomers may be obtained.

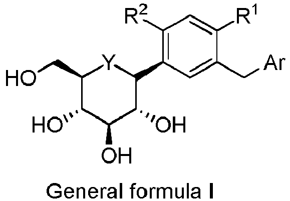

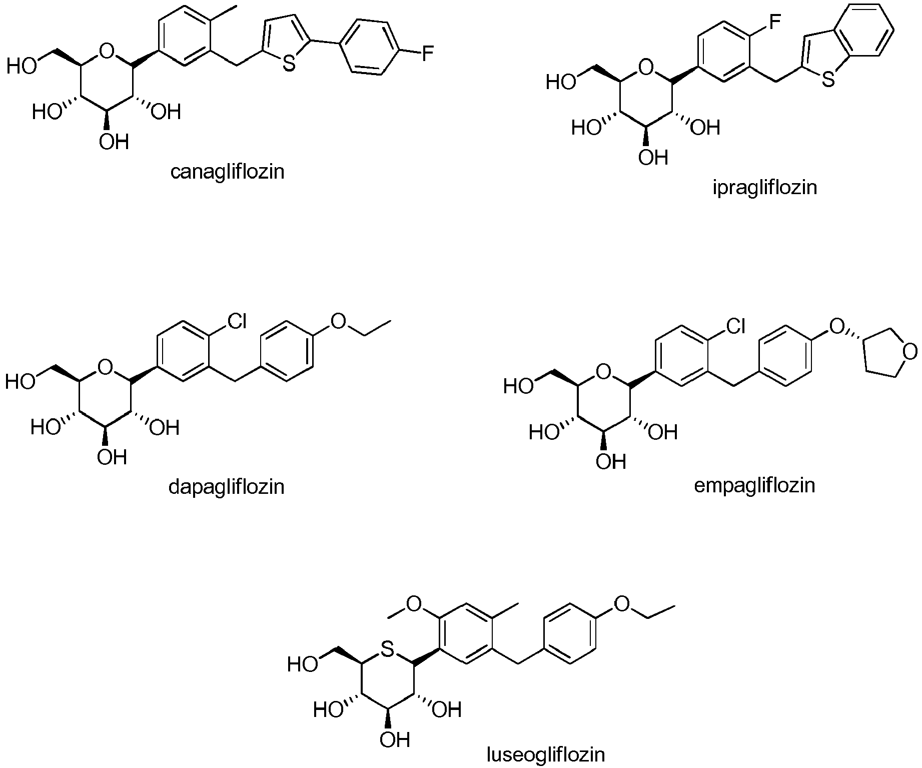

Several methods are known in the art for the synthesis of β-C-arylglycosides such as canagliflozin. For example, WO 2005/012326 generally describes a class of C-glycoside compounds including canagliflozin to inhibit the sodium-dependent glucose transporter (SGLT) and thus a therapeutic use for treatment of diabetes, obesity, diabetic complications, and the like. Moreover, the patent application describes a process for the preparation of said C-glycoside compounds, however solely *via* coupling of protected gluconolactone with the aryl/heteroaryl moiety, followed by subsequent reduction and deprotection. Since the compound may be obtained in the form of a diastereomer or enantiomer, one has to separate the diastereomer or enantiomer of interest by conventional methods such as chromatography or fractional crystallization.

Another process for the preparation of canagliflozin is shown in WO 2010/043862**.** This patent application describes a coupling between protected gluconolactone and Grignard derivative prepared from appropriate iodo heterocycle derivative, followed by reduction and deprotection. Furthermore, this document describes the subsequent acetylation and recrystallization of tetraacetyl substituted canagliflozin for purification purposes.

WO 2011/142478 expands the process described in WO 2010/43862, wherein contrary thereto the coupled intermediate is first acetylated followed by reduction and final deprotection of the tetraacetyl substituted canagliflozin.

WO 2008/69327 is directed to a process for the preparation of preferred hydrates, solvates and crystalline forms of canagliflozin.

WO 2012/140120 describes the process of coupling using organozinc intermediates prepared from a iodo substituted heterocycle, an alkyl lithium substance and a tetrapivaloyl protected bromo-glucose derivative. Zinc is used in equimolar amounts in order to form intermediate bisarylzinc intermediate.

WO 2013/068850 describes process of preparation β-C-arylglycosides by coupling of 2,4-di-O-protected 1,6-anhydroglucopyranose or 2,3,4-tri-O-protected 1,6-anhydroglucopyranose derivatives with nucleophilic aryl compounds.

US 2007/0073046 mentions an option of 1-arylation of glucopyranosides via 1,2-epoxides, but the article does not describe any experimental results and therefore it does not show results of *cis*/*trans* selectivity. The cited reference in this publication **(**Tetrahedron 58, 1997-2009 (2002)) only shows unpredictability of *cis*/*trans* selectivity which depends on reagents catalyst and substituents.

Taking into account the synthesis of gliflozins and particularly of canagliflozin and related compounds as known in prior art there is a need, and hence it is an object of the present invention, to provide a stereoselective and efficient synthesis of β-gliflozins, such as canagliflozin, as well as to provide useful intermediate compounds in the desired *trans* configuration at relatively low costs.

These objects as well as others, which will become apparent from the following description of the present invention, are attained by the subject-matter of the independent claims. Some of the preferred embodiments of the present invention are defined by the subject matter of the dependent claims.

### Summary of the Invention

Various aspects, advantageous features and preferred embodiments of the present invention as summarized in the following items, respectively alone or in combination, contribute to solving the object of the invention.
1. A process for the preparation of a compound of formula 3', 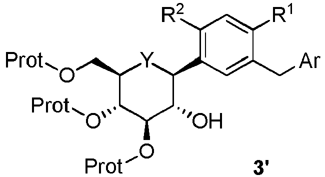
   wherein Y represents O or S,
   R¹ represent halo, C₁₋₆-alkyl or C₁₋₆-alkoxy,
   R² represent hydrogen, halo, C₁₋₆-alkyl or C₁₋₆-alkoxy,
   Ar represents a phenyl, thienyl, or benzothienyl group, which is optionally substituted by one or more groups selected from halo, C₁₋₆-alkyl or OR', wherein R' is selected from C₁₋₆-alkyl, C₅₋₇-cycloalkyl or oxa-C₅₋₇-cycloalkyl and,
   Prot represents a protection group,
   said process comprising the steps of:
      a) coupling an aryl derivate of formula 2' 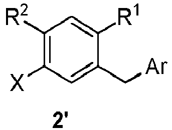 wherein X represents a group that can react with or can be exchanged with a metal or a metal containing group, which is attached to the phenyl group by a metal atom;
      b) coupling the compound of formula 2' to a protected glucal epoxide of the formula **1'** 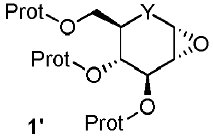
2. The process according to item **1,** wherein the aryl derivate of the formula **2'** is activated with said metal by a reaction with an elemental metal, a metal salt or an organometal reagent; and
   wherein the protected glucal epoxide of the formula 1' is coupled to the activated compound of formula **2'.**
3. The process according to item 1 or 2, wherein
   a) the compound of formula 3' is a compound of formula 3 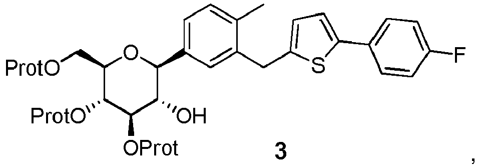
   b) the compound of formula 2' is a compound of formula **2** 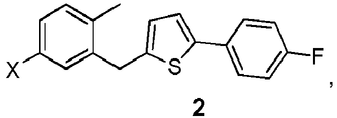 and
   c) the compound of formula 1' is a compound of formula **1** 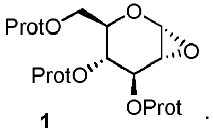
4. The process according to item 4, wherein X is Br, I, or Cl, and preferably wherein X is Br.
5. The process according to any of the preceding items, wherein the metal is magnesium, lithium, copper, aluminum, titanium, palladium, or zinc, or wherein the metal is contained in a preparation such as Grignard reagent with elementary magnesium, sec-butylmagnesium chloride lithium chloride complex, isopropylmagnesium chloride lithium chloride complex or a preparation of magnesate with reagents such as tributyl lithium or isopropyllithium magnesate or their derivatives.
6. The process according to any of the preceding items, wherein the protection group is selected from an ether forming group, selected from mono or poly aryl substituted alkyl such as benzyl or substituted benzyl such as para-methoxybenzyl, diphenylmethyl, trityl; ester forming groups selected from unsubstituted or substituted aliphatic or aromatic acyl groups such as acetyl, pivaloyl, benzoyl, or alkyl or aryl substituted silyl groups such as trimethylsilyl, *tert*-butyldimethylsilyl.
7. The process according to any of the preceding items, wherein the protection group is benzyl, para-methoxybenzyl, or pivaloyl.
8. The process according to any of the preceding items, comprising the addition of copper salt, the copper salt being neat or in solution.
9. The process according to item 8, wherein the copper salt is selected from the group consisting of CuCN, CuI, CuBr, CuCl, CuSCN, preferably CuCN.
10. The process according to items 8 or 9, comprising the addition of a metal salt, preferably a lithium salt, more preferably a lithium halide, most preferably LiCl, the metal salt being able to form a complex with the copper salt.
11. The process according to item 10, wherein the complex with the copper salt is CuCN·2LiCl.
12. The process according to any of the items 8 to 11, wherein the copper salt is added catalytically in an amount from 0.1 to 0.5 mole per 1 mole of the compound **2'** or **2.**
13. The process according to any of items 8 to 12, wherein the copper salt is added to the aryl derivate of the formula **2', 2,** preferably after the aryl derivate has been activated with the metal according to item 2.
14. The process according to any of the items 8 to 13, wherein the copper salt is added to the protected epoxide of the formula **1', 1** with subsequent addition of the activated aryl compound of the formula **2', 2.**
15. The process according to item 14, wherein the activated aryl compound of the formula **2', 2** is added slowly to the protected epoxide of the formula **1', 1,** preferably in distinct portions.
16. The process according to any of the items 8 to 15, wherein the activated aryl derivate is forming with copper salt a diarylcuprate compound Ar¹Ar²Cu represented by the formula 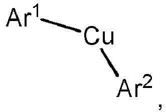 and/or a cuprate complex Ar¹Ar²Cu(X')M₂ represented by the formula 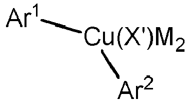 with an accompanied anion X', X' being selected from CN, I, Br, Cl, or SCN, and a metal cation M, preferably lithium,
   wherein Ar¹ and Ar² are the same or different and respectively correspond to the structural moiety of formula **2', 2** bound via X.
17. The process according to item 16, wherein Ar¹ and Ar² correspond to the structural moiety of formula **2'** bound via X to provide a diarylcuprate complexes of the formula **7'** and/or **7A',** 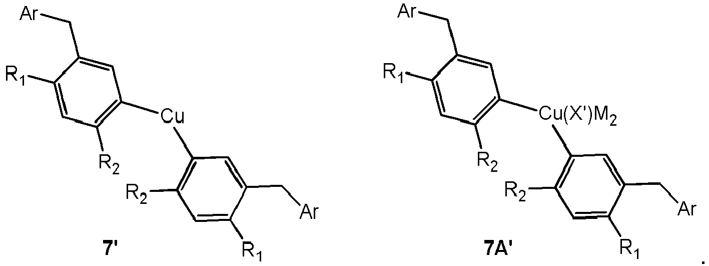
18. The process according to item 16, wherein Ar¹ and Ar² correspond to the structural moiety of formula **2** bound via X to provide a diarylcuprate complexes of the formula **7** and/or **7A,** 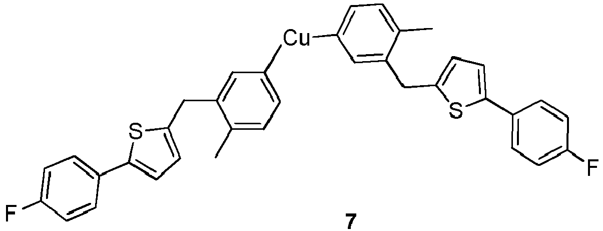 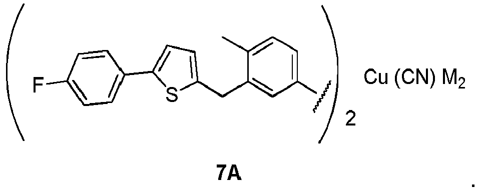
19. The process according to any of items 8 to 18, wherein the copper salt is added in a range from approximately equimolarly to approximately catalytically.
20. The process according to any of the items 8 to 19, further comprising the addition of at least one additional ligand A, the ligand being able to form mixed organocuprates to provide a compound of formula **4B',** 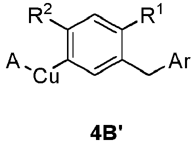 preferably higher order mixed organocuprates with the aryl derivate, wherein A represents an organic radical and/or ligand, and wherein Ar, R¹ and R² are defined as above.
21. The process according to item 20, wherein the at least one additional ligand A is selected from the group consisting of thienyl, butoxy, and phenylmercapto.
22. A process for the preparation of a compound of formula **I** 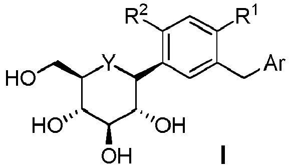 or a pharmaceutically acceptable complex or solvate thereof, comprising the step(s) according to any of the preceding items, and further comprising the step of deprotecting the compound of formula **3'.**
23. The process according to item 22, wherein the compound of formula **I** is canagliflozin with formula **8** 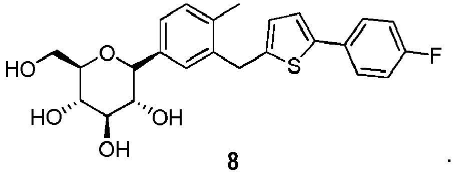
24. The process according to any of the preceding items, further comprising at least one purification step.
25. The process according to any of the preceding items, further comprising at least one quenching step.
26. A process for the preparation of a pharmaceutical composition comprising a compound of formula **I** or a pharmaceutically acceptable complex or solvate thereof, and a pharmaceutically acceptable carrier and/or excipient, the process comprising carrying out a process according to any of the items 1 to 25 for preparing an intermediate compound of formula **3',** a step of deprotecting the intermediate compound of formula **3'** to obtain the compound of formula I, and a step of mixing the compound of formula **I** with the pharmaceutically acceptable carrier and/or excipient 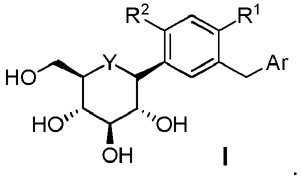
27. The process according to item 26 for the preparation of a pharmaceutical composition comprising canagliflozin or a pharmaceutically acceptable salt or solvate thereof, and a pharmaceutically acceptable carrier and/or excipient.
28. A diarylcuprate compound Ar¹Ar²Cu represented by formula 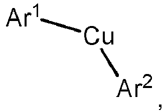 and/or a diarylcuprate complex compound Ar¹Ar²Cu(X')M₂ represented by the formula 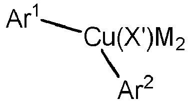 wherein Ar¹ and Ar² are the same or different and respectively correspond to the following structural moiety **2B'** 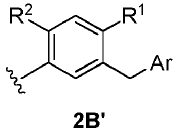
   wherein R¹ represents halo, C₁₋₆-alkyl or C₁₋₆-alkoxy,
   R² represents hydrogen, halo, C₁₋₆-alkyl or C₁₋₆-alkoxy,
   Ar represents a phenyl, thienyl, or benzothienyl group, which is optionally substituted by one or more groups selected from halo, C₁₋₆-alkyl or OR', wherein R' is selected from C₁₋₆-alkyl, C₅₋₇-cycloalkyl or oxa-C₅₋₇-cycloalkyl, and
   wherein X' is an accompanied anion and is selected from CN, I, Br, Cl, or SCN, preferably CN and M is a metal cation, preferably lithium.
29. The compound of item 28, wherein at least one aryl derivate Ar¹ or Ar² corresponds to the following structural moiety of formula **2B** 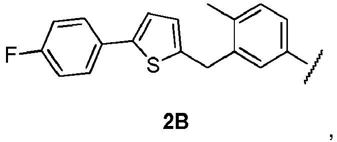 X' is CN and M is lithium.
30. A mixed organocuprate compound Ar¹ACu represented by the formula 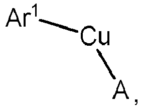 and/or a mixed organocuprate complex compound Ar¹ACu(X')M₂ represented by the formula 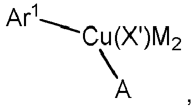 wherein Ar¹ corresponds to the following structural moiety **2B'** 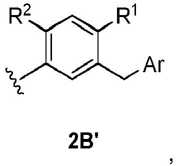 wherein R¹ represents halo, C₁₋₆-alkyl or C₁₋₆-alkoxy, R² represents hydrogen, halo, C₁₋₆-alkyl or C₁₋₆-alkoxy, Ar represents a phenyl, thienyl, or benzothienyl group, which is optionally substituted by one or more groups selected from halo, C₁₋₆-alkyl or OR', wherein R' is selected from C₁₋₆-alkyl, C₅₋₇-cycloalkyl or oxa-C₅₋₇-cycloalkyl, wherein X' is an accompanied anion and is selected from CN, I, Br, Cl, or SCN, and M is a metal cation, preferably lithium, and wherein A is an additional ligand, the ligand being able to form mixed organocuprates, optionally higher order mixed organocuprates with the aryl derivate.
31. The compound of item 30, wherein the aryl derivate Ar¹ corresponds to the following structural moiety of formula **2B** 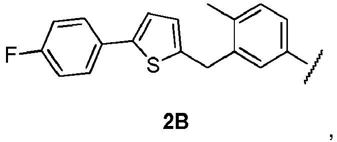 and wherein X' is CN, M is lithium and A is selected from the group consisting of thienyl, butoxy, and phenylmercapto.
32. A mixture comprising the cuprate compound according to any of items 28 to 31 and an epoxide compound of the formula 1' in a solvent, 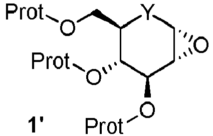 wherein Y represents O or S, and Prot represents a protection group.
33. The mixture according to item 32, wherein Y represents O.
34. A compound of the formula 3', 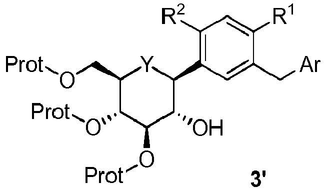 wherein Y represents O or S,
   R¹ represents halo, C₁₋₆-alkyl or C₁₋₆-alkoxy,
   R² represents hydrogen, halo, C₁₋₆-alkyl or C₁₋₆-alkoxy,
   Ar represents a phenyl, thienyl, or benzothienyl group, which is optionally substituted by one or more groups selected from halo, C₁₋₆-alkyl or OR', wherein R' is selected from C₁₋₆-alkyl, C₅₋₇-cycloalkyl or oxa-C₅₋₇-cycloalkyl and,
   Prot represents a protection group.
35. The compound according to item 34, defined by formula 3 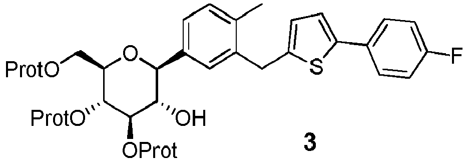
36. Use of a compound or of a mixture according to any of the items 28 to 35 as an intermediate in the preparation of a gliflozin compound of the formula **I.**
37. Use according to item 36 in the preparation of canagliflozin of the formula **8.**

### Definitions

**Alkyl:** organic group derived from an alkane

**C-glycoside:** a carbohydrate derivative, including a glucose derivative, wherein the carbohydrate is bound to a non-carbohydrate moiety and the carbohydrate is bound to the non-carbohydrate moiety via a carbon-carbon covalent bond

**Arylglycoside:** the carbohydrate of the C-glycoside is bound to an aromatic moiety via a carbon-carbon covalent bond

**β-configuration:** As used herein, the prefix α- and β- refer to the configuration of the anomeric center of the C-(aryl)glycoside. In the β -C-arylglycoside, the aryl group (i.e., the aglycone) is in the same relative position with respect to the other chemical bonds at the anomeric center as the hydroxy group is in β-glucose. In this configuration, the aryl group on C-1 is in a ***trans*** configuration to the hydroxy group of C-2.

### Detailed description of the invention

The present invention is now described in more detail by preferred embodiments and examples, which are however presented for illustrative purpose only and shall not be understood as limiting the scope of the present invention in any way.

The present invention provides an industrially applicable, economical and advantageous process for the preparation of intermediates of compound of formula **3',** 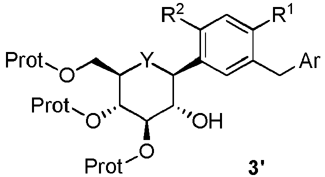
wherein Y represent O or S,
R¹ represents halo, C₁₋₆-alkyl or C₁₋₆-alkoxy,
R² represents hydrogen, halo, C₁₋₆-alkyl or C₁₋₆-alkoxy,
Ar represents a phenyl, thienyl, or benzothienyl group, which is optionally substituted by one or more groups selected from halo, C₁₋₆-alkyl or OR', wherein R' is selected from C₁₋₆-alkyl, C₅₋₇-cycloalkyl or oxa-C₅₋₇-cycloalkyl and,
Prot represents a protection group.

Compound of formula **3'** represents a valuable intermediate for preparing pharmaceutically active agents, preferably sodium-dependent glucose cotransporter 2 (SGLT2) inhibitors such as gliflozins, particularly canagliflozin or structurally similar gliflozins, such as ipragliflozin, empagliflozin, dapagliflozin and luseogliflozin.

The preparation process according to the invention starts from the protected glucal epoxide compound of formula **1'** and a suitable substituted aryl derivate of the formula **2'.** Surprisingly, it was found by the inventors that the aforementioned compounds are able to couple involving the coupling group X according to scheme 1 to a product according to formula **3'** with a remarkable stereoselectivity in favor of the desired *trans*-isomer. The stereoselectivity in favor of the desired trans-isomer may be higher than 5.5:1, preferably higher than 20:1, even up to 50:1 or higher. Moreover, it was surprisingly found, that the aforementioned reaction takes place efficiently, i.e. with up to quantitative conversion into the product.

For example, the coupling group X may represent a group that may be exchanged with a metal or a metal containing group, that group being preferably a halogen such as a Br, I, or Cl. Any protection group suitable for epoxides may be selected, for example from an ether forming group, selected from mono or poly aryl substituted alkyl such as benzyl or substituted benzyl such as para-methoxybenzyl, diphenylmethyl, trityl; an ester forming groups selected from unsubstituted or substituted aliphatic or aromatic acyl groups such as acetyl, pivaloyl, benzoyl, or alkyl or aryl substituted silyl groups such as trimethylsilyl, *tert*-butyldimethylsilyl In a preferred example the protecting groups are the same in all three hydroxy group.

A general concept of the process of the present invention is presented in Scheme 1, wherein Prot represents any suitable protection group, X represents any coupling group, and Y, R¹, R² and Ar are as defined above.

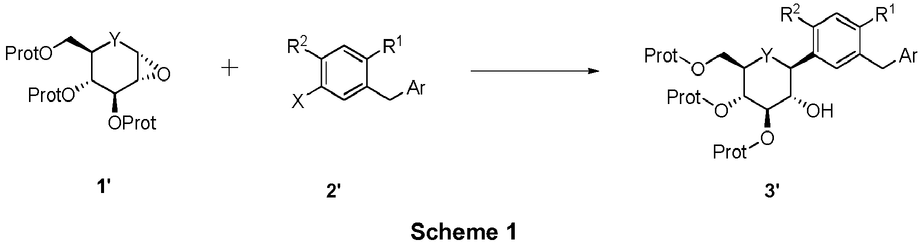

In a preferred embodiment of the present invention, X can be chosen from groups, which can be transformed to more reactive organometal intermediates by a reaction with an elemental metal, such as lithium or magnesium, by anhydrous metal salts, selected but not limited to lithium, magnesium, aluminum, zinc, titanium, copper (I), palladium halogenides, cyanides, alkoxides or triflates or organometal compounds such as alkyl lithium, Grignard reagents or a combination thereof. In a more preferred embodiment of the present invention, wherein X may be Br, I or Cl, the corresponding compound of formula **2'** is transformed to intermediate of general formula **4,** wherein M* represents a metal containing group wherein metal is directly attached to the phenyl core by a single bond. Depending on valence and properties of a particular metal the intermediates can be represented by formulae **4A, 4B, 4C, 4D** or **4E,** wherein M is selected but not limited to magnesium, lithium, copper, aluminum, titanium, palladium, or zinc, Z or Z' can represent one or more accompanied anions, another same or different metal atoms, or organic radicals or ligands and wherein the bond between M and Z/Z' is covalent, ionic or coordinative.

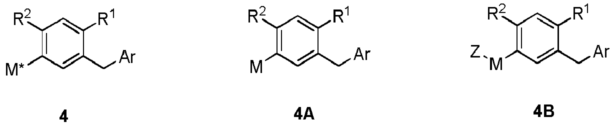

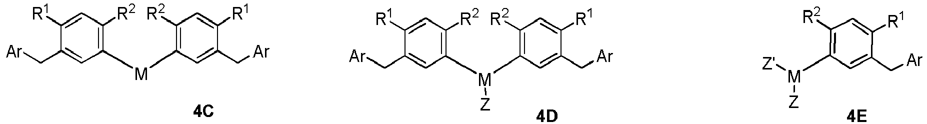

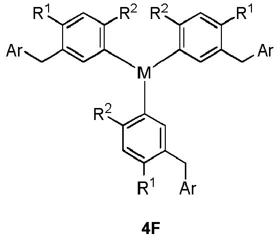

For example, if the metal is monovalent the formula **4** can be represented by special structures **4A** or **4B,** wherein constituent parts of Z and/or Z' are bound by coordinative bonds, if metal is divalent the formula **4** can be represented by special structures **4B, 4C, 4D,** or **4E,** wherein one of constituent parts of Z/Z' is not bound by a coordinative bond, and if the metal is trivalent the formula **4** can be represented by special structures **4B, 4D, 4E,** or **4F,** wherein remaining valences are consumed by non-coordinative bonds with constituent parts of Z/Z'. And if the complex of formula **4** is charged it needs an additional counterion. The intermediates of the formula **4** are "activated" forms of the preferred embodiments of the compounds of the formula **2'** prepared for the coupling according to the invention. They do not need to be isolated and preferably they are actually not isolated, but they are used in a solution for further transformations. The further transformations as used herein mean a direct coupling with the epoxide of formula **1, 1'** or a preparation of a still more reactive or more selective reagent for the coupling the said epoxide. Such advanced intermediates can be prepared by addition of another metal containing reagent in excess, equimolar or catalytic amounts. Optionally, the intermediates of the formula **4** and their more advanced forms are short living species, which are generated just before the reaction with the epoxide.

In representative examples of the general concept the compound of formula **2'** is selected from the compounds **2A, 2D, 2E, 2F** and **2G.** These compounds are suitable starting materials for the synthesis of canagliflozin, ipragliflozin, empagliflozin, luseogliflozin, and dapagliflozin following the process of the present invention.

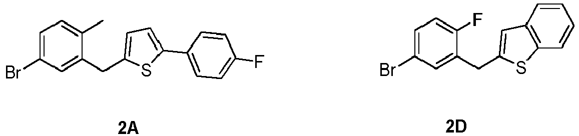

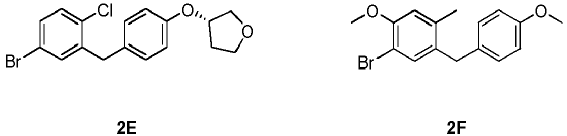

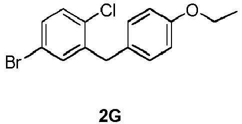

In a more specific example of the general concept of the process of the present invention a preparation of canagliflozin can be described in further detail below by reference to **Scheme 2,** which illustratively represents the general concept according to preferred embodiments, wherein Prot represents any suitable protection group and X any coupling group X. In a preferred embodiment of the present invention, X can be chosen from groups which may be reacted or exchanged with metals such as, but not limited to, magnesium, lithium, copper, aluminum, titanium, palladium, zinc, and so on. In a more preferred embodiment of the present invention X may be Br, I or Cl. In a most preferred embodiment of the present invention X may be Br.

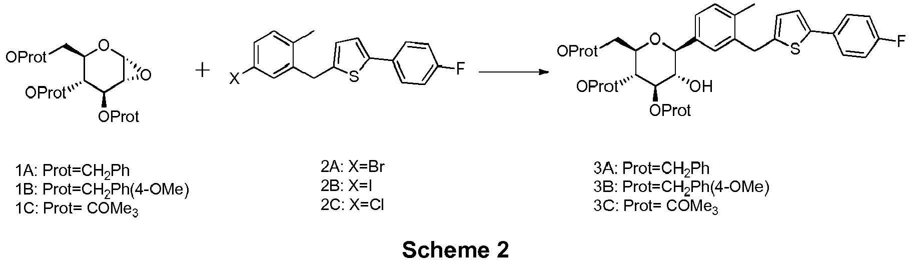

Firstly, the compound of formula **2'** is for example activated as known in the art by substituting the coupling group X with a metal in an appropriate solvent, the metal being for example magnesium, lithium, copper, aluminum, titanium, palladium, zinc or similar or the metal being comprised in a reagent, for example a Grignard reagent, e.g. with elementary magnesium, with other Grignard reagents such as sec-butylmagnesium chloride lithium chloride complex, isopropylmagnesium chloride lithium chloride complex or preparation of magnesate with reagents such as tributyl lithium or isopropyllithium magnesate or their derivatives as already known in the art. The reaction solvent may be an aprotic solvent, preferably a polar aprotic solvent, for example THF.

In a first aspect, the thus activated aryl derivate is added to a solution of protected glucal epoxide in a suitable solvent, preferably an aprotic solvent, more preferably a polar-aprotic solvent, for example THF, at a temperature ranging from around -80°C to 120°C, preferable from around 0°C to 25°C. The addition might be either slow or rapid. The reaction mixture is then stirred at a selected temperature range until completion, followed by purifying and/or de-protecting the product using standard procedures to give rise to the compound of general formula **I, 8.**

In a process according to the invention it was surprisingly found that the protected glucal epoxide of the formula **1', 1** effectively and efficiently reacts with the activated aryl compound of the formula **2', 2** to give rise to a novel intermediate compound of formula **3', 3.** Moreover, it was found that the process proceeds with a remarkable stereoselectivity in favour of the desired isomer having the aryl moiety at C-1 of the glucal ring, the aryl moiety being in a *trans* configuration to the hydroxyl group formed at C-2 of the glucal ring according to formula **3', 3,** which is the preferred intermediate compound in the synthesis of gliflozins such as canagliflozin or structurally similar gliflozins, such as ipragliflozin and other related compounds. Although not wishing to be bound by any theory, this may at least be due to the steric effects in regard of the "chair" configuration and of the protection group on position C-3 on the glucal epoxide and the epoxide three-membered ring, allowing the activated aryl compound to attack the epoxide from that side of the glucal ring with the lowest steric barriers. Hence, a beneficial intermediate product according to formula **3', 3** is formed having the hydroxy group on C-2 of the glucal ring, i.e. next to the protection group on position C-3 and preferably being in a *trans* configuration thereto. Consequently, this may be one of the reasons why the activated aryl group is preferably coupled to C-1 of the glucal ring and is preferably coupled in a *trans* configuration relative to the hydroxyl group of C-2 of the glucal ring.

After activation of the compound **2** with lithium or magnesium metal the configuration *trans* is favored but still considerable amounts of *cis* isomer or even 1,2-regioisomer may be found found. For example, in one special experiment a Grignard reagent, prepared from the compound **2** is transformed by direct coupling with the epoxide of formula **1A** to the main *trans* product of formula **3A,** but combined with the *cis* isomer of formula **5A** and the 1,2-regioisomer according to formula **6A** (Example 8, **Scheme 4).**

It was surprisingly found that when preferably copper(I) salt is added, the amount of the *cis* isomer distinctively reduced under 4%, preferably under 2%, more preferably under 1 % in comparison to *trans* isomer with regard to the total mixture after reaction, while the regioisomer is reduced to undetectable amounts. In such preferred case three metals participate in the coupling, lithium, magnesium and copper.

In a further preferred embodiment, at least one copper(I) salt may be added to the solution of aryl derivate activated as described above. The copper salt may be neat or in solution in an anhydrous aprotic solvent, e.g. THF and may be selected from compounds such as CuCN, CuI, CuBr, CuCl, CuSCN or similar inorganic or organic copper salts. In a standard procedure the compound of the formula **2', 2** is first activated with lithium-magnesium complexes, such as tributyllithium magnesate, followed by addition of a copper reagent, preferably prepared a copper(I) salt and a lithium salt, preferably lithium halide. Preferred copper salts for such reagent are CuI, CuSCN and CuCN, most preferred is CuCN; the preferred lithium salt is LiCl. The copper(I) salt and the lithium salt may be added as a complex, preferably as CuCN·2LiCl. The thus prepared organocopper reagent is added to the solution of protected epoxide at temperature ranging from -80°C to 120°C, preferable from 0°C to 25°C. The addition might be either slow or rapid. The reaction mixture is then stirred at a selected temperature range until completion, followed by purifying and/or deprotecting the product using standard procedures to give rise to the compound of formula **I, 8.**

The thus prepared organocopper reagent is complex, since it is predominately consists of a diarylcuprate compound (Ar¹Ar²Cu) and/or a cuprate complex with an accompanied anion X' and a metal cation, such as lithium, wherein the complex Ar¹Ar²Cu(X')M₂, wherein X' is selected from CN, I, Br, Cl, or SCN, wherein M is the metal cation, preferably Li, and Ar¹ and Ar² are the same or different and at least one of them derives from the compound of formula **2'.**

In an embodiment where no additional organometal reagent is added, the complex is symmetrical, wherein Ar¹ and Ar² are the same and the radical Ar¹/Ar² has the origin in the compound of formula **2'.** In such case at least two moles of the compound **2'** are consumed in the reaction and only one equivalent is involved in the coupling. The symmetrical intermediates are represented by formulae **7'** and **7A',** M being the metal cation, preferably Li.

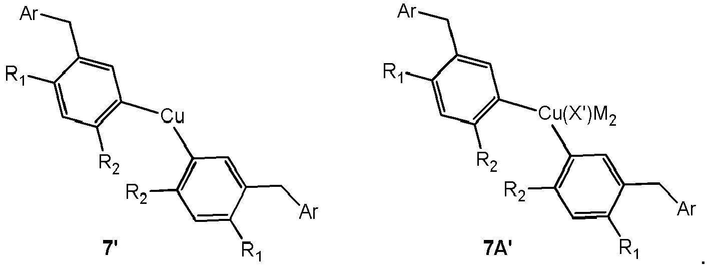

*Trans*/*cis* selectivity of the said procedure is good and a yield of the *cis* isomers **(5', 5)** falls under 4%, preferably under 1%, while the whole transformation is not complete, presumably due to a longstanding instability of the organocopper complex. It was surprisingly found that the formation of the complex is very fast with enabling *in situ* formation of such complex just before coupling.

Thus, in a preferred embodiment, the copper reagent is primarily added to glucal epoxide followed by a subsequent addition of lithium/magnesium activated polycyclic compound of formula **4** at a temperature range from -80°C to 120°C, preferable from 0 to 25°C. Preferably, the lithium/magnesium activated polycyclic compound of formula **4** is added slowly to the mixture of copper reagent and glucal epoxide, for example in portions and/or dropwise. The short living *in situ* forming organocuprate complex of formula **7'/7A'** quickly reacts with glucal epoxide. The reaction is then stirred at the selected temperature until completion, followed by purifying and/or deprotecting the product using standard procedures to give rise to the compound of formula **3', 3.** Such improved process elevates yield for 25-50 % with remaining high *trans*/*cis* selectivity.

Furthermore, applying such reversed mixing of reagents the amount of the copper salt may be reduced to catalytical quantities. An amount of a copper salt in the catalytic procedure is 0.01 mole to 0.99 mole per 1 mole of the compound **2'** or **2,** preferably from 0.1 to 0.5 mole per 1 mole. A reduced use of copper salts lowers production costs and what is more important it reduces copper waste. Such process is more industry and environment friendly.

The protocol according to the second aspect may be expanded by adding additional ligands in the cuprate preparation step to form higher order mixed organocuprates with the aryl moiety. Such mixed organocuprate compounds can be described by a general formula Ar¹ACu or Ar¹ACu(X')M₂ in case of mixed organocuprate complexes. More specifically the mixed organocuprate compounds can be described by formula **4B',** wherein A represents an organic radical/ligand, and Ar, R¹, and R² are defined as above. The additional ligand A is selected from, but not limited to be thienyl, butoxy, or phenylmercapto. The mixed organocuprates are, for example, prepared using the corresponding lithium reagents such as thienyl lithium, lithium butoxide, lithium thiophenolate, respectively. The activated (di)arylcopper intermediate of formula **4** is unsymmetrical, wherein one expensive part, which derives from the compound of formula **2** is replaced by a cheaper organic moiety. The consumption of the compound of formula **2** is equimolar in this case.

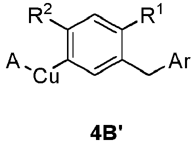

In a special example of this aspect of the invention a mixed organocuprate of the formula **7B** is prepared by addition of thiophene and butyllithium to the previously prepared solution CuCN·2LiCl complex followed by addition of the compound of the formula **2A.**

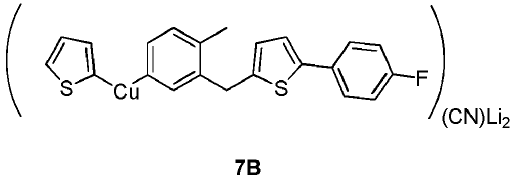

The compound of formula 3' prepared according to the procedure of the present invention is finally deprotected by conventional methods to give compounds of general formula I, which are potent sodium-dependent glucose cotransporter 2 (SGLT2) inhibitors. For example, benzyl or substituted benzyl group can be removed by catalytic hydrogenation or by use of silyl iodide decoupling, while the pivaloyl group can be deprotected by acidic hydrolytic conditions.

The following examples further illustrate the invention. They are provided for illustrative purposes only and are not intended to limit the invention in any way.

### Experimental procedures

### Example 1: Synthesis of (2R,3S,4R,5R,6R)-4,5-bis(benzyloxy)-6-((benzyloxy)methyl)-2-(3-((5-(4-fluorophenyl)thiophen-2-yl)methyl)-4-methylphenyl)tetrahydro-2H-pyran-3-ol (formula 3A, scheme 3)

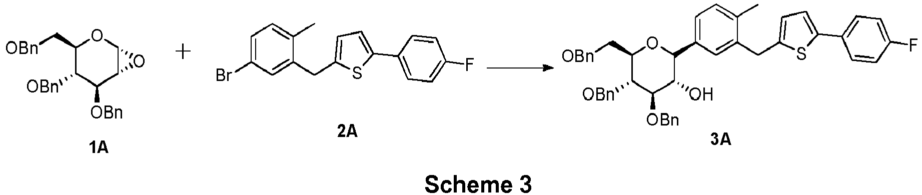

Under argon atmosphere, 2-(5-bromo-2-methylbenzyl)-5-(4-fluorophenyl)thiophene **(2A,** 10.8 g) was dissolved in THF (40 ml) and cooled to 0°C. To this solution, 15 ml of tributyllithium magnesate solution (0.7M in ether/hexanes) was slowly added and left at 0°C for 20 minutes. At 0°C 15 ml of copper cyanide dilithiumchloride complex (1 M) was added and left at 0°C for 30 min. To reaction mixture a solution of (1S,3R,4R,5S,6R)-4,5-bis(benzyloxy)-3-((benzyloxy)methyl)-2,7-dioxabicyclo[4.1.0]heptane **(1A,** 6.49 g) in THF (15 ml) was slowly added. After complete addition, reaction mixture was left to warm up to r.t. and left overnight to give the mixture containing 29% *trans* isomer (2R,3S,4R,5R,6R)-4,5-bis(benzyloxy)-6-((benzyloxy)methyl)-2-(3-((5-(4-fluorophenyl)thiophen-2-yl)methyl)-4-methylphenyl)tetrahydro-2H-pyran-3-ol **(3A)** and 1.6% of the *cis* isomer corresponding to the trans-isomer of formula **3A** (HPLC). Reaction mixture might be further purified using flash column chromatography (ethyl acetate/heptane 1:6). MS; M+NH₄⁺ = 732.

### Example 2: Synthesis of (2R,3S,4R,5R,6R)-4,5-bis(benzyloxy)-6-((benzyloxy)methyl)-2-(3-((5-(4-fluorophenyl)thiophen-2-yl)methyl)-4-methylphenyl)tetrahydro-2H-pyran-3-ol (formula 3A, scheme 3)

Under argon atmosphere, 2-(5-bromo-2-methylbenzyl)-5-(4-fluorophenyl)thiophene **(2A,** 3.61g) was dissolved in THF (10 ml) and cooled to 0°C. To this solution 4.76 ml of tributyllithium magnesate solution (0.7M in ether/hexanes) was slowly added and left at 0°C for 20 minutes. At 0°C 5 ml of copper cyanide dilithiumchloride complex (1M) was added and left at 0°C for 30 min. To reaction mixture a solution of (1S,3R,4R,5S,6R)-4,5-bis(benzyloxy)-3-((benzyloxy)methyl)-2,7-dioxabicyclo[4.1.0]heptane **(1A,** 1.95 g) in THF (5 ml) was slowly added. After complete addition reaction mixture was left to warm up to r. t. and left overnight to give the mixture containing (2R,3S,4R,5R,6R)-4,5-bis(benzyloxy)-6-((benzyloxy)methyl)-2-(3-((5-(4-fluorophenyl)thiophen-2-yl)methyl)-4-methylphenyl) tetrahydro-2H-pyran-3-ol **(3A).** Reaction mixture might be further purified using flash column chromatography (ethyl acetate/heptane 1:6). MS; M+NH₄⁺ = 732.

### Example 3: Synthesis of (2R,3S,4R,5R,6R)-4,5-bis(benzyloxy)-6-((benzyloxy)methyl)-2-(3-((5-(4-fluorophenyl)thiophen-2-yl)methyl)-4-methylphenyl)tetrahydro-2H-pyran-3-ol (formula 3A, scheme 3)

Under argon atmosphere, 2-(5-bromo-2-methylbenzyl)-5-(4-fluorophenyl)thiophene **(2A,** 2.16 g) was dissolved in THF (10 ml) and cooled to 0°C. To this solution 2.9 ml of tributyllithium magnesate solution (0.7M in ether/hexanes) was slowly added and left at 0°C for 20 minutes. At 0°C 3 ml of copper cyanide dilithiumchloride complex (1M) was added and left at 0°C for 30 min. To reaction mixture a solution of (1S,3R,4R,5S,6R)-4,5-bis(benzyloxy)-3-((benzyloxy)methyl)-2,7-dioxabicyclo[4.1.0]heptane **(1A,** 1.30 g) in THF (15 ml) was slowly added. After complete addition reaction mixture was left to warm up to r. t. and left overnight to give the mixture containing 20% *trans* isomer (2R,3S,4R,5R,6R)-4,5-bis(benzyloxy)-6-((benzyloxy)methyl)-2-(3-((5-(4-fluorophenyl)thiophen-2-yl)methyl)-4-methylphenyl)tetrahydro-2H-pyran-3-ol (3A) and 1% of the *cis* isomer corresponding to the trans-isomer of formula **3A** (HPLC). Reaction mixture might be further purified using flash column chromatography (ethyl acetate/heptane 1:6). MS; M+NH₄⁺ = 732.

### Example 4: Synthesis of (2R,3S,4R,5R,6R)-4,5-bis(benzyloxy)-6-((benzyloxy)methyl)-2-(3-((5-(4-fluorophenyl)thiophen-2-yl)methyl)-4-methylphenyl)tetrahydro-2H-pyran-3-ol (formula 3A, scheme 3)

Under argon atmosphere, 2-(5-bromo-2-methylbenzyl)-5-(4-fluorophenyl)thiophene **(2A,** 2.16g) was dissolved in THF (10 ml) and cooled to 0°C. To this solution 8.6 ml of tributyllithium magnesate solution (0.7M in ether/hexanes) was slowly added and left at 0°C for 20 minutes. At 0°C 3 ml of copper cyanide dilithiumchloride complex (1M) was added and left at 0°C for 30 min. To reaction mixture a solution of (1S,3R,4R,5S,6R)-4,5-bis(benzyloxy)-3-((benzyloxy)methyl)-2,7-dioxabicyclo[4.1.0]heptane **(1A,** 1.30 g) in THF (15 ml) was slowly added. After complete addition reaction mixture was left to warm up to r. t. and left overnight to give the mixture containing 22% *trans* isomer (2R,3S,4R,5R,6R)-4,5-bis(benzyloxy)-6-((benzyloxy)methyl)-2-(3-((5-(4-fluorophenyl)thiophen-2-yl)methyl)-4-methylphenyl)tetrahydro-2H-pyran-3-ol **(3A)** and 4% of the *cis* isomer corresponding to the *trans* isomer of formula **3A** (HPLC). Reaction mixture might be further purified using flash column chromatography (ethyl acetate/heptane 1:6). MS; M+NH₄⁺ = 732.

### Example 5: Synthesis of (2R,3S,4R,5R,6R)-4,5-bis(benzyloxy)-6-((benzyloxy)methyl)-2-(3-((5-(4-fluorophenyl)thiophen-2-yl)methyl)-4-methylphenyl)tetrahydro-2H-pyran-3-ol (formula 3A, scheme 3)

Under argon atmosphere, 2-(5-bromo-2-methylbenzyl)-5-(4-fluorophenyl)thiophene **(2A,** 4.33 g) was dissolved in THF (15 ml) and cooled to 0°C. To this solution 6 ml of tributyllithium magnesate solution (0.7M in ether/hexanes) was slowly added and left at 0°C for 20 minutes. This solution was slowly (20 min) added to a solution of ( S,3R,4R,5S,6R)-4,5-bis(benzyloxy)-3-((benzyloxy)methyl)-2,7-dioxabicyclo[4.1.0] heptane **(1A,** 4.71 g) and copper cyanide dilithiumchloride complex (1M, 1.1 ml) in THF (8 ml). The mixture was stirred at r. t. for several hours (72.4% *trans* **3A;** then quenched with saturated ammonium chloride, stirred at r. t. for another hour then extracted with ethyl acetate. Organic phase was further purified using flash column chromatography (ethyl acetate/heptane 1:6) to give 3.42 g of pure (HPLC 99.4 area%) (2R,3S,4R,5R,6R)-4,5-bis(benzyloxy)-6-((benzyloxy)methyl)-2-(3-((5-(4-fluorophenyl)thiophen-2-yl)methyl)-4-methylphenyl)tetrahydro-2H-pyran-3-ol **(3A)** (44%). MS; M+NH₄⁺ = 732.

### Example 6: Synthesis of (2R,3S,4R,5R,6R)-4,5-bis(benzyloxy)-6-((benzyloxy)methyl)-2-(3-((5-(4-fluorophenyl)thiophen-2-yl)methyl)-4-methylphenyl)tetrahydro-2H-pyran-3-ol (formula 3A, scheme 3)

Under argon atmosphere, 2-(5-bromo-2-methylbenzyl)-5-(4-fluorophenyl)thiophene **(2A,** 4.13 g) was dissolved in THF (15 ml) and cooled to 0°C. To this solution 5.7 ml of tributyllithium magnesate solution (0.7M in ether/hexanes) was slowly added and left at 0°C for 20 minutes. This solution was slowly (20 min) added to a solution of ( S,3R,4R,5S,6R)-4,5-bis(benzyloxy)-3-((benzyloxy)methyl)-2,7-dioxabicyclo[4.1.0] heptane **(1A,** 4.71 g) and copper cyanide dilithiumchloride complex (1 M, 0.544 ml) in THF (8 ml). The mixture was stirred at r. t. for several hours (76% *trans* **3A),** then quenched with saturated ammonium chloride, stirred at r. t. for another hour then extracted with ethyl acetate. Organic phase was further purified using flash column chromatography (ethyl acetate/heptane 1:6) to give 4.92 g of pure (HPLC 99.0 area%) (2R,3S,4R,5R,6R)-4,5-bis(benzyloxy)-6-((benzyloxy)methyl)-2-(3-((5-(4-fluorophenyl)thiophen-2-yl)methyl)-4-methylphenyl)tetrahydro-2H-pyran-3-ol **(3A)** (63%). MS; M+NH₄⁺ = 732.
NMR (1 H, DMSO): 2.28 (s, 3H), 3.41-3.67 (m, 6H); 4.09-4-13 (m, 3H), 4.45-4.54 (m, 3H), 4.73-4.76 (m, 2H); 4.94 (d, 1H); 5.22 (d, 1H), 6.80 (d, 1H), 7.16-7.31 (m, 19H), 7.36 (m, 2H), 7.56-7.58 (m, 2H).

### Example 7: Synthesis of (2R,3S,4R,5R,6R)-4,5-bis(benzyloxy)-6-((benzyloxy)methyl)-2-(3-((5-(4-fluorophenyl)thiophen-2-yl)methyl)-4-methylphenyl)tetrahydro-2H-pyran-3-ol (formula 3A, scheme 3)

Under argon atmosphere, 2-(5-bromo-2-methylbenzyl)-5-(4-fluorophenyl)thiophene **(2A,** 4.03 g) was dissolved in THF (15 ml) and cooled to 0°C. To this solution 5.6 ml of tributyllithium magnesate solution (0.7M in ether/hexanes) was slowly added and left at 0°C for 20 minutes. This solution was slowly (20 min) added to a solution of ( S,3R,4R,5S,6R)-4,5-bis(benzyloxy)-3-((benzyloxy)methyl)-2,7-dioxabicyclo[4.1.0] heptane **(1A,** 4.71 g) and copper cyanide dilithiumchloride complex (1 M, 0.272 ml) in THF (8 ml). The mixture was stirred at r. t. for several hours (65% *trans* 3A), then quenched with saturated ammonium chloride, stirred at r. t. for another hour then extracted with ethyl acetate. Organic phase was further purified using flash column chromatography (ethyl acetate/heptane 1:6) to give 4.02 g of pure (HPLC 98.9 area%) (2R,3S,4R,5R,6R)-4,5-bis(benzyloxy)-6-((benzyloxy)methyl)-2-(3-((5-(4-fluorophenyl)thiophen-2-yl)methyl)-4-methylphenyl)tetrahydro-2H-pyran-3-ol **(3A)** (52).MS; M+NH₄⁺ = 732.

### Example 8: Synthesis of (2R,3S,4R,5R,6R)-4,5-bis(benzyloxy)-6-((benzyloxy)methyl)-2-(3-((5-(4-fluorophenyl)thiophen-2-yl)methyl)-4-methylphenyl)tetrahydro-2H-pyran-3-ol, the corresponding cis isomer and regiomer (formulas 4, 5, 6, scheme 4)

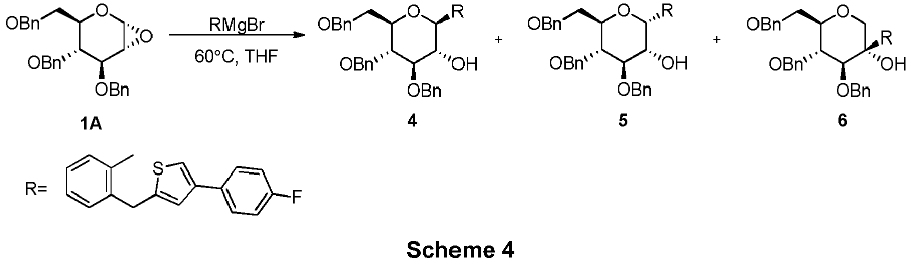

The freshly prepared (3-((5-(4-fluorophenyl)thiophen-2-yl)methyl)-4-methylphenyl) magnesium bromide (0.28 M in THF, 5 mL, 1.4 mmol) was added dropwise to a solution of ( S,3R,4R,5S,6R)-4,5-bis(benzyloxy)-3-((benzyloxy)methyl)-2,7-dioxabicyclo[4.1.0] heptane (0.5 g, 1.16 mmol) in THF (1.2 mL) at 60 °C. The reaction mixture was stirred for 1.5 hours then quenched with saturated NH₄Cl solution and diluted with EtOAc. After phase separation, aqueous phase was extracted three times with EtOAc (20 mL). The combined organic layers were washed with brine (30 mL), dried over MgSO₄, filtered and concentrated in vacuo. Purification by column chromatography (heptane:EtOAc=6:1) afforded pure product (0.48 g, 58%) as a mixture of *trans*/*cis* isomers (4:5>9:1) and regioisomers (4:6=1.6:1).

### Example 9: Synthesis of canagliflozin (CANA, (2S,3R,4R,5S,6R)-2-{3-[5-[4-fluorophenyl)-thiophen-2-ylmethyl]-4-methyl-phenyl}-6-hydroxymethyl-tetrahydro-pyran-3,4,5-triol) starting from (2R,3S,4R,5R,6R)-4,5-bis(benzyloxy)-6-((benzyloxy)methyl)-2-(3-((5-(4-fluorophenyl)thiophen-2-yl)methyl)-4-methylphenyl)tetrahydro-2H-pyran-3-ol (scheme 5)

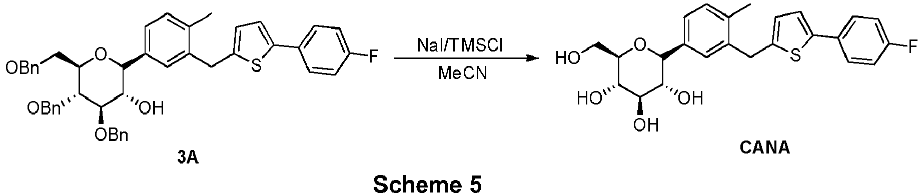

0.52 g (2R,3S,4R,5R,6R)-4,5-bis(benzyloxy)-6-((benzyloxy)methyl)-2-(3-((5-(4-fluoro-phenyl)thiophen-2-yl)methyl)-4-methylphenyl)tetrahydro-2H-pyran-3-ol (3A) was dissolved in absolute acetonitrile (4 ml). 0.65 g of sodium iodide was added, solution was cooled to 0°C and 0.55 ml of trimethylsilyl chloride was slowly (10 min) added. Suspension was left to warm up to ambient temperature, stirred from another 60 min then quenched with saturated sodium thiosulfate (20 ml), extracted with ethyl acetate (3x30 ml) washed with thiosulfate and brine, then dried and concentrated. Hexane (20 ml) was added to the oily residue and stirred until white precipitate formed. The latter was collected with filtration to give canagliflozin (0.31 g).

### Example 10: Synthesis of (2S,3S,4R,5R,6R)-2-(3-((5-(4-fluorophenyl)thiophen-2-yl)methyl)-4-methylphenyl)-4,5-bis((4-methoxybenzyl)oxy)-6-(((4-methoxybenzyl)oxy)methyl)tetrahydro-2H-pyran-3-ol (formula 3B, scheme 6)

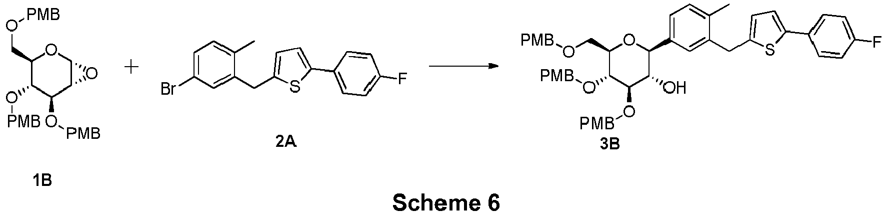

Under argon atmosphere, 2-(5-bromo-2-methylbenzyl)-5-(4-fluorophenyl)thiophene **(2A,** 3.25 g) was dissolved in THF (10 ml) and cooled to 0°C. To this solution 4.28 ml of tributyllithium magnesate solution (0.7M in ether/hexanes) was slowly added and left at 0°C for 20 minutes. At 0°C 5 ml of copper cyanide dilithiumchloride complex (1M) was added and left at 0°C for 30 min. To reaction mixture a solution of (1S,3R,4R,5S,6R)-4,5-bis((4-methoxybenzyl)oxy)-3-(((4-methoxybenzyl)oxy)methyl)-2,7-dioxabicyclo[4.1.0] heptane (**1B**, 2.09 g) in THF (5 ml) was slowly added. After complete addition reaction mixture was left to warm up to r. t. and left overnight to give the mixture containing (2S,3S,4R,5R,6R)-2-(3-((5-(4-fluorophenyl)thiophen-2-yl)methyl)-4-methylphenyl)-4,5-bis((4-methoxybenzyl)oxy)-6-(((4-methoxybenzyl)oxy)methyl)tetrahydro-2H-pyran-3-ol (**3B**). Reaction mixture was further purified using flash column chromatography (ethyl acetate/cyclohexane to give 0.82 g of crude material, which was suspended in methylcyclohexane (10 ml), stirred for several hours then filtered to give 0.50 g of pure (HPLC 98.48 area%) material. MS; M+NH₄⁺ = 822. NMR (1 H, DMSO): 2.27 (s, 3H); 3.34-3.59 (m, 6H), 3.68 (s, 3H), 3.71 (s, 3H), 3.72 (s, 3H), 4.05-4.12 (m, 3H), 4.34-4.44 (m, 3H), 4.62-4.68 (m, 2H), 4.84 (d, 1 H), 5.16 (d, 1 H), 6.79 (d, 1 H), 6.82-6.86 (m, 6H), 7.04 (d, 2H), 7.14-7.29 (m, 10H), 7.55-7.58 (m, 2H).

### Example 11: Synthesis of (2R,3R,4R,5S,6S)-6-(3-((5-(4-fluorophenyl)thiophen-2-yl)methyl)-4-methylphenyl)-5-hydroxy-2-((pivaloyloxy)methyl)tetrahydro-2H-pyran-3,4-diyl bis(2,2-dimethylpropanoate) (formula 3C, scheme 7)

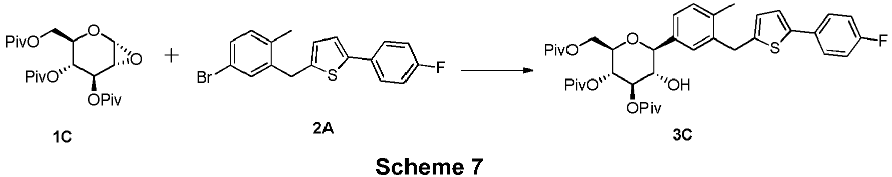

Under argon atmosphere, 2-(5-bromo-2-methylbenzyl)-5-(4-fluorophenyl)thiophene **(2A,** 3.61 g) was dissolved in THF (10 ml) and cooled to 0°C. To this solution 4.76 ml of tributyllithium magnesate solution (0.7M in ether/hexanes) was slowly added and left at 0°C for 20 minutes. At 0°C 5 ml of copper cyanide dilithiumchloride complex (1M) was added and left at 0°C for 30 min. To reaction mixture a solution of (1S,3R,4R,5S,6R)-3-((pivaloyloxy)methyl)-2,7-dioxabicyclo[4.1.0]heptane-4,5-diyl bis(2,2-dimethylpropanoate) **(1C,** 1.87 g) in THF (5 ml) was slowly added. After complete addition reaction mixture was left to warm up to r. t. and left overnight to give the mixture containing (2R,3R,4R,5S,6S)-6-(3-((5-(4-fluorophenyl)thiophen-2-yl)methyl)-4-methylphenyl)-5-hydroxy-2-((pivaloyloxy)methyl)tetrahydro-2H-pyran-3,4-diyl bis(2,2-dimethylpropanoate) (3C). Reaction mixture might be further purified using flash column chromatography (ethyl acetate/heptane 1:6). MS; M+NH₄⁺ = 714.

### Example 12: Synthesis of (2R,3S,4R,5R,6R)-4,5-bis(benzyloxy)-6-((benzyloxy)methyl)-2-(3-((5-(4-fluorophenyl)thiophen-2-yl)methyl)-4-methylphenyl)-tetrahydro-2H-pyran-3-ol using equimolar ratio of 2A and 1 B (scheme 8)

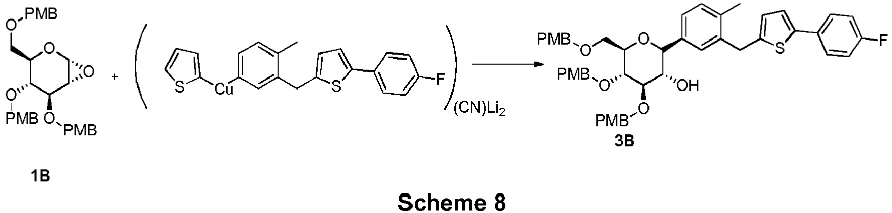

Under argon atmosphere at 0°C, to a solution of copper cyanide dilithiumchloride complex (1M, 3 ml) a solution prepared at -80°C from thiophene (243 µl) in THF (5 ml) and butyllithium (1.6M, 1.9 ml) was slowly added. After that, a solution prepared at 0°C from 2-(5-bromo-2-methylbenzyl)-5-(4-fluorophenyl)thiophene **(2A,** 1.08 g) in THF and 4.3 ml of tributyllithium magnesate solution (0.7M in ether/hexanes), matured for 20 min, was slowly added. To this mixture a solution of (1S,3R,4R,5S,6R)-4,5-bis((4-methoxybenzyl)oxy)-3-(((4-methoxybenzyl)oxy)methyl)-2,7-dioxabicyclo[4.1.0]heptane (**1B**, 1.31 g in THF (5 ml) was slowly added, left to warm up to r. t. and left overnight to give the mixture containing 6% of *trans* isomer (2S,3S,4R,5R,6R)-2-(3-((5-(4-fluorophenyl)thiophen-2-yl)methyl)-4-methylphenyl)-4,5-bis((4-methoxybenzyl)oxy)-6-(((4-methoxybenzyl)oxy)methyl) tetrahydro-2H-pyran-3-ol (**3B**). Reaction mixture might be further purified using flash column chromatography (ethyl acetate/heptane 1:6). MS; M+NH₄⁺ = 822

### Example 13: Synthesis of (2S,3S,4R,5R,6R)-2-(3-(benzo[b]thiophen-2-ylmethyl)-4-fluorophenyl)-4,5-bis(benzyloxy)-6-((benzyloxy)methyl)tetrahydro-2H-pyran-3-ol (formula 3D, scheme 9):

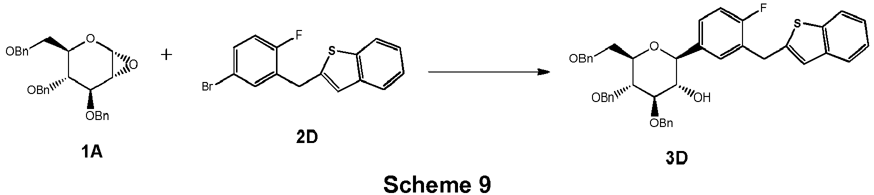

Under argon atmosphere, 2-(5-bromo-2-fluorobenzyl)benzo[b]thiophene **(2D,** 3.58 g) is dissolved in THF (15 ml) and cooled to 0°C. To this solution 5.6 ml of tributyllithium magnesate solution (0.7M in ether/hexanes) is slowly added and left at 0°C for 20 minutes. This solution is slowly (20 min) added to a solution of (1S,3R,4R,5S,6R)-4,5-bis(benzyloxy)-3-((benzyloxy)methyl)-2,7-dioxabicyclo[4.1.0]heptane **(1A,** 4.71 g) and copper cyanide dilithiumchloride complex (1M, 0.272 ml) in THF (8 ml). The mixture is stirred at r.t for several hours, then quenched with saturated ammonium chloride, stirred at r. t. for another hour then extracted with ethyl acetate. Organic phase is further purified using flash column chromatography (ethyl acetate/heptane 1:6) to give (2S,3S,4R,5R,6R)-2-(3-(benzo[b]thiophen-2-ylmethyl)-4-fluorophenyl)-4,5-bis(benzyloxy)-6-((benzyloxy) methyl)tetrahydro-2H-pyran-3-ol **(3D).**

### Example 14: Synthesis of (2S,3S,4R,5R,6R)-4,5-bis(benzyloxy)-6-((benzyloxy) methyl)-2-(4-chloro-3-(4-(((S)-tetrahydrofuran-3-yl)oxy)benzyl)phenyl)tetrahydro-2H-pyran-3-ol (formula 3E, scheme 10):

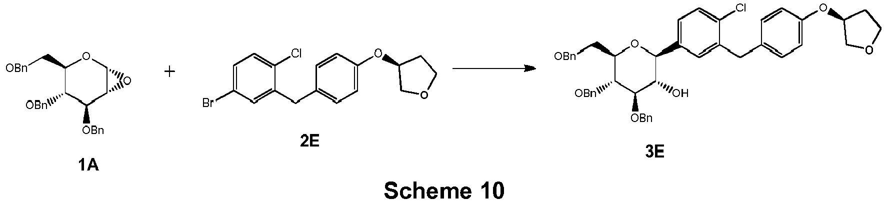

Under argon atmosphere (S)-3-(4-(5-bromo-2-chlorobenzyl)phenoxy)tetrahydrofuran **(2E,** 4.10 g) is dissolved in THF (15 ml) and cooled to 0°C. To this solution 5.6 ml of tributyllithium magnesate solution (0.7M in ether/hexanes) is slowly added and left at 0°C for 20 minutes. This solution is slowly (20 min) added to a solution of (1S,3R,4R,5S,6R)-4,5-bis(benzyloxy)-3-((benzyloxy)methyl)-2,7-dioxabicyclo[4.1.0]heptane **(1A,** 4.71g) and copper cyanide dilithiumchloride complex (1M, 0.272 ml) in THF (8 ml). The mixture is stirred at r. t. for several hours, then quenched with saturated ammonium chloride, stirred at r. t. for another hour then extracted with ethyl acetate. Organic phase is further purified using flash column chromatography (ethyl acetate/heptane 1:6) to give (2S,3S,4R,5R,6R)-4,5-bis(benzyloxy)-6-((benzyloxy)methyl)-2-(4-chloro-3-(4-(((S)-tetrahydrofuran-3-yl)oxy)benzyl)phenyl)tetrahydro-2H-pyran-3-ol **(3E).**

### Example 15: Synthesis of (2S,3S,4R,5R,6R)-4,5-bis(benzyloxy)-6-((benzyloxy)-methyl)-2-(4-chloro-2-methoxy-5-(4-methoxybenzyl)phenyl)tetrahydro-2H-pyran-3-ol (formula 3F, scheme 11):

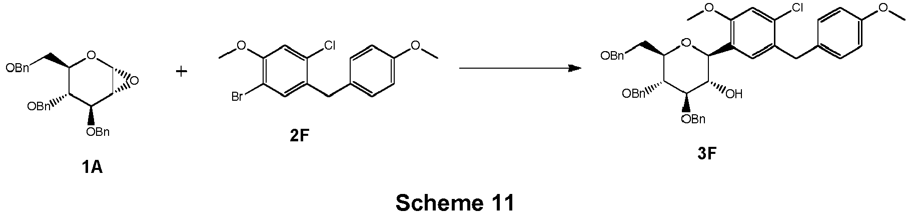

Under argon atmosphere 1-bromo-4-chloro-2-methoxy-5-(4-methoxybenzyl)benzene **(2F,** 3.81 g) is dissolved in THF (15 ml) and cooled to 0°C. To this solution 5.6 ml of tributyllithium magnesate solution (0.7M in ether/hexanes) is slowly added and left at 0°C for 20 minutes. This solution is slowly (20 min) added to a solution of (1S,3R,4R,5S,6R)-4,5-bis(benzyloxy)-3-((benzyloxy)methyl)-2,7-dioxabicyclo[4.1.0]heptane **(1A,** 4.71 g) and copper cyanide dilithiumchloride complex (1M, 0.272 ml) in THF (8 ml). The mixture is stirred at r. t. for several hours, then quenched with saturated ammonium chloride, stirred at r. t. for another hour then extracted with ethyl acetate. Organic phase is further purified using flash column chromatography (ethyl acetate/heptane 1:6) to give (2S,3S,4R,5R,6R)-4,5-bis(benzyloxy)-6-((benzyloxy)methyl)-2-(4-chloro-2-methoxy-5-(4-methoxybenzyl) phenyl)tetrahydro-2H-pyran-3-ol (3F).

### Example 16: Synthesis of (2S,3S,4R,5R,6R)-4,5-bis(benzyloxy)-6-((benzyloxy)-methyl)-2-(4-chloro-3-(4-ethoxybenzyl)phenyl)tetrahydro-2H-pyran-3-ol (formula 3G, scheme 12)

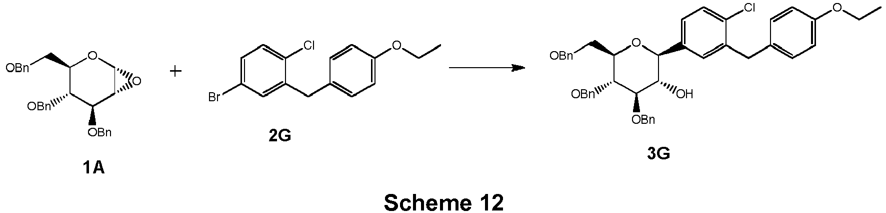

Under argon atmosphere 4-bromo-1-chloro-2-(4-ethoxybenzyl)benzene **(2G,** 3.64 g) is dissolved in THF (15 ml) and cooled to 0°C. To this solution 5.6 ml of tributyllithium magnesate solution (0.7M in ether/hexanes) is slowly added and left at 0°C for 20 minutes. This solution is slowly (20 min) added to a solution of (1S,3R,4R,5S,6R)-4,5-bis(benzyloxy)-3-((benzyloxy)methyl)-2,7-dioxabicyclo[4.1.0]heptane **(1A,** 4.71 g) and copper cyanide dilithiumchloride complex (1M, 0.272 ml) in THF (8 ml). The mixture is stirred at r. t. for several hours, then quenched with saturated ammonium chloride, stirred at r. t. for another hour then extracted with ethyl acetate. Organic phase is further purified using flash column chromatography (ethyl acetate/heptane 1:6) to give (2S,3S,4R,5R,6R)-4,5-bis(benzyloxy)-6-((benzyloxy)methyl)-2-(4-chloro-3-(4-ethoxy-benzyl)phenyl) tetrahydro-2H-pyran-3-ol **(3G).**

## Claims

1. A process for the preparation of a compound of formula 3', 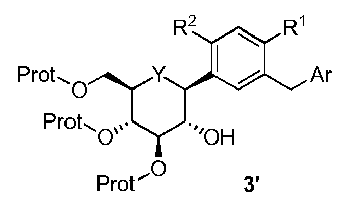
wherein Y represents O or S,
R¹ represents halo, C₁₋₆-alkyl or C₁₋₆-alkoxy,
R² represents hydrogen, halo, C₁₋₆-alkyl or C₁₋₆-alkoxy,
Ar represents a phenyl, thienyl, or benzothienyl group, which is optionally substituted by one or more groups selected from halo, C₁₋₆-alkyl or OR', wherein R' is selected from C₁₋₆-alkyl, C₅₋₇-cycloalkyl or oxa-C₅₋₇-cycloalkyl and,
Prot represents a protection group,
said process comprising the steps of:
a) coupling an aryl derivate of the formula 2' 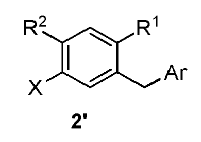 wherein X represents a group that can react with or can be exchanged with a metal or a metal containing group, which is attached to the phenyl group by a metal atom;
b) coupling the compound of formula **2'** to a protected glucal epoxide of the formula 1'

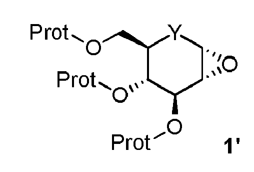

2. The process according to claim 1,
wherein the aryl derivate of the formula **2'** is activated with said metal by a reaction with an elemental metal, a metal salt or an organometal reagent; and
wherein the protected glucal epoxide of the formula 1' is coupled to the activated compound of formula **2'.**

3. The process according to claim 1 or claim 2, wherein
a) the compound of formula **3'** is compound of formula **3** 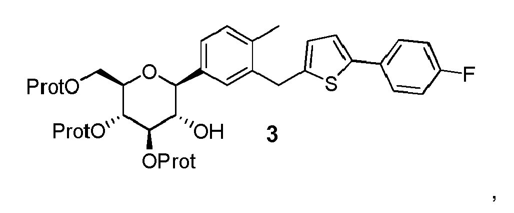
b) the compound of formula **2'** is compound of formula **2** 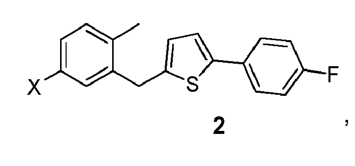 and
c) the compound of formula **1'** is compound of formula **1**

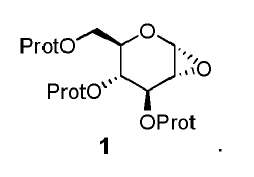

4. The process according to any one of the preceding claims, wherein X is Br, I, or Cl, preferably Br.

5. The process according to any one of the preceding claims, wherein the metal is magnesium, lithium, copper, aluminum, titanium, palladium, or zinc, or wherein the metal is contained in a preparation selected from a Grignard reagent with elementary magnesium, sec-butylmagnesium chloride lithium chloride complex, isopropylmagnesium chloride lithium chloride complex or a preparation of magnesate with reagents, preferably tributyl lithium or isopropyllithium magnesate or their derivatives.

6. The process according to any one of the preceding claims, further comprising the addition of a copper salt, the copper salt being neat or in solution and/or being a copper complex, preferably CuCN·2LiCl.

7. The process according to claim 6, wherein the copper salt is added to the protected epoxid of the formula **1', 1** with subsequent addition of the activated aryl compound of formula **2', 2,** preferably wherein the activated aryl compound of formula **2', 2** is added slowly.

8. The process according to claim 7, wherein the copper salt is added catalytically in an amount from 0.1 to 0.5 mole per 1 mole of the compound **2'** or **2.**

9. The process according to any one of claims 6 to 8, further comprising the addition of at least one additional ligand A, the ligand being able to form mixed organocuprates, preferably higher order mixed organocuprates with the aryl derivate to provide a compound of formula **4B',** 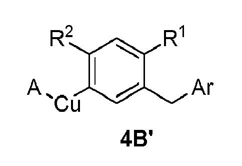 wherein A represents an organic radical and/or ligand, preferably thienyl, butoxy, and phenylmercapto, and Ar, R¹ and R² are defined as above.

10. A process for the preparation of a compound of the formula I 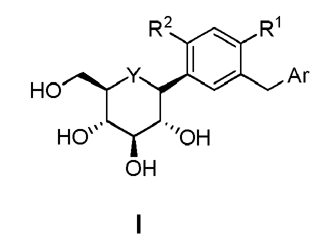 or a pharmaceutically acceptable complex or solvate thereof,
wherein Y represents O or S,
R¹ represents halo, C₁₋₆-alkyl or C₁₋₆-alkoxy,
R² represents hydrogen, halo, C₁₋₆-alkyl or C₁₋₆-alkoxy,
Ar represents a phenyl, thienyl, or benzothienyl group, which is optionally substituted by one or more groups selected from halo, C₁₋₆-alkyl or OR', wherein R' is selected from C₁₋₆-alkyl, C₅₋₇-cycloalkyl or oxa-C₅₋₇-cycloalkyl,
comprising the step(s) according to any of the preceding claims, and further comprising the step of de-protecting the compound of formula 3'

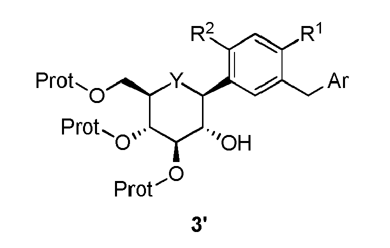

11. The process according to claim 10, wherein the compound of formula **I** is canagliflozin of formula 8

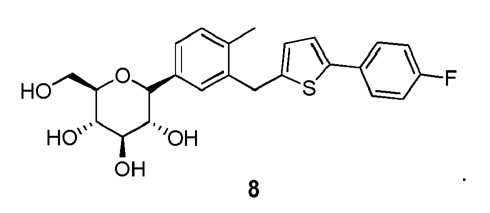

12. A process for the preparation of a pharmaceutical composition comprising a gliflozin compound of the formula **I** or a pharmaceutically acceptable complex or solvate thereof, 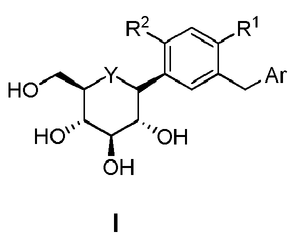
wherein Y represents O or S,
R¹ represents halo, C₁₋₆-alkyl or C₁₋₆-alkoxy,
R² represents hydrogen, halo, C₁₋₆-alkyl or C₁₋₆-alkoxy,
Ar represents a phenyl, thienyl, or benzothienyl group, which is optionally substituted by one or more groups selected from halo, C₁₋₆-alkyl or OR', wherein R' is selected from C₁₋₆-alkyl, C₅₋₇-cycloalkyl or oxa-C₅₋₇-cycloalkyl,
the process comprising a process according to any of the claims 1 to 11 for preparing an intermediate compound of formula **3'** 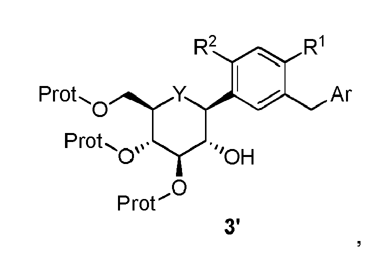 a step of de-protecting the intermediate compound of formula 3', and a step of mixing the compound of formula **I** with the pharmaceutically acceptable carrier and/or excipient.

13. A diarylcuprate compound Ar¹Ar²Cu represented by formula 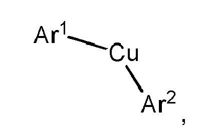 and/or a diarylcuprate complex compound Ar¹Ar²Cu(X')M₂ represented by the formula 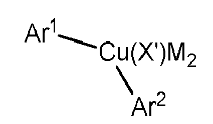 wherein Ar¹ and Ar² are the same or different and respectively correspond to the following structural moiety **2B'** 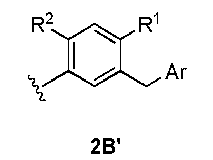
wherein R¹ represents halo, C₁₋₆-alkyl or C₁₋₆-alkoxy,
R² represents hydrogen, halo, C₁₋₆-alkyl or C₁₋₆-alkoxy,
Ar represents a phenyl, thienyl, or benzothienyl group, which is optionally substituted by one or more groups selected from halo, C₁₋₆-alkyl or OR', wherein R' is selected from C₁₋₆-alkyl, C₅₋₇-cycloalkyl or oxa-C₅₋₇-cycloalkyl, and
wherein X' is an accompanied anion and is selected from CN, I, Br, Cl, or SCN, preferably CN and M is a metal cation, preferably lithium.

14. A mixed organocuprate compound Ar¹ACu represented by the formula 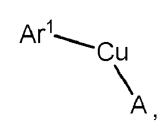 and/or a mixed organocuprate complex compound Ar¹ACu(X')M₂ represented by the formula 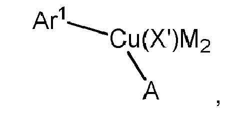 wherein Ar¹ corresponds to the following structural moiety **2B'** 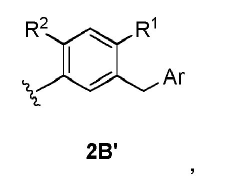
wherein R¹ represents halo, C₁₋₆-alkyl or C₁₋₆-alkoxy,
R² represents hydrogen, halo, C₁₋₆-alkyl or C₁₋₆-alkoxy,
Ar represents a phenyl, thienyl, or benzothienyl group, which is optionally substituted by one or more groups selected from halo, C₁₋₆-alkyl or OR', wherein R' is selected from C₁₋₆-alkyl, C₅₋₇-cycloalkyl or oxa-C₅₋₇-cycloalkyl,
wherein X' is an accompanied anion and is selected from CN, I, Br, Cl, or SCN, and M is a metal cation, preferably lithium, and
wherein A is an additional ligand, the ligand being able to form mixed organocuprates, optionally higher order mixed organocuprates with the aryl derivate.

15. A mixture comprising the cuprate compound according to claim 13 or claim 14 and an epoxide compound of the formula 1' in a solvent, 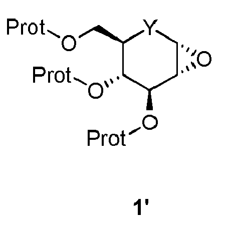 wherein Y represents O or S, and Prot represents a protection group.

16. A compound of the formula 3', 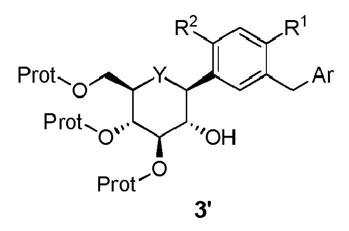
wherein Y represents O or S,
R¹ represents halo, C₁₋₆-alkyl or C₁₋₆-alkoxy,
R² represents hydrogen, halo, C₁₋₆-alkyl or C₁₋₆-alkoxy,
Ar represents a phenyl, thienyl, or benzothienyl group, which is optionally substituted by one or more groups selected from halo, C₁₋₆-alkyl or OR', wherein R' is selected from C₁₋₆-alkyl, C₅₋₇-cycloalkyl or oxa-C₅₋₇-cycloalkyl, and
Prot represents a protection group.

17. The compound of claim 16, defined by formula 3

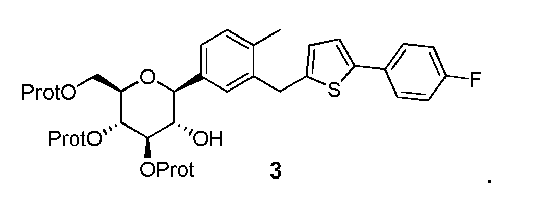

18. Use of a compound or a mixture according to any of the claims 13 to 17 as an intermediate in the preparation of a gliflozin compound of the formula I 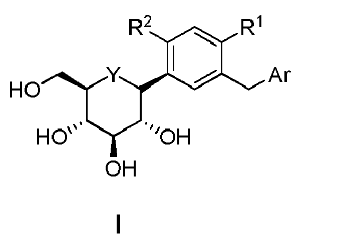 or a pharmaceutically acceptable complex or solvate thereof.
